# EUROPEAN PATENT APPLICATION

(11) **EP 4 729 087 A1**
(43) Date of publication of application: **22.04.2026**
(21) Application number: 24207531.5
(22) Date of filing: 18.10.2024
(51) Int. Cl.: A61M 5/142, A61M 5/145, A61M 5/168

(54) **PATCH TYPE INSULIN PUMP AND INSULIN INJECTION SYSTEM**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application relates to the technical field of medical appliances and discloses a patch type insulin pump and an insulin injection system and its method of application. The patch type insulin pump comprises an injection module, a power module and an injection detection module. The injection module comprises a drug storage tank, a push rod piston and two conductive push rods. The bottom of the drug storage tank is provided with an injection port, and the top is provided with an opening. The push rod piston is sealed on the opening, and the push rod piston may move relative to the drug storage tank along a first direction. The two conductive push rods are respectively connected to the push rod piston. The injection detection module comprises a control circuit board, two conductive contact springs and a conductive module. The two conductive contact springs are electrically connected to the control circuit board, and the two conductive push rods are in a sliding contact with the two conductive contact springs. The power module is used to drive the conductive module to move in the first direction, and the control circuit board is used to detect the dosage injected into the drug storage tank based on the moving distance of the conductive module. The patch type insulin pump can detect the dosage injected by the patch type insulin pump.

## Description

### Technical Field

The present application relates to the technical field of medical appliances, and in particular to a patch type insulin pump and an insulin injection system.

### Background

At present, the patch type insulin pump can achieve automatic drug injection and bolus. To ensure the accurate dosage injected by users, users need to inject the insulin liquid from the standard drug bottle into the drug storage tank of the patch type insulin pump in advance according to the instructions before injection. However, due to human errors, users may not follow the instructions when injecting medication into the drug storage tank, resulting in insufficient dosing (a. medicine leaked at the needle and was not completely injected into the medicine storage tank; b. the scale of the syringe was worn or missing, and the dose of inhaled medicine was insufficient; c. the medicine storage tank was accidentally damaged and the medicine flowed out) or overdosing in reference to the suggested dosage by the doctor, affecting the treatment effect. At present, the existing patch type insulin pumps do not have the ability to detect the injected dosage, which affects the user experience.

### Summary

The present application provides a patch type insulin pump and an insulin injection system, capable of detecting the dosage injected by the patch type insulin pump, so that users can use medication accurately.

In a first aspect, the present application provides a patch type insulin pump, comprising an injection module, a power module and an injection detection module;
wherein the injection module comprises a drug storage tank, a push rod piston, and two conductive push rods; the bottom of the drug storage tank is provided with an injection port, and the top of the drug storage tank is provided with an opening; the push rod piston is sealed on the opening, and the push rod piston can move relative to the drug storage tank along a first direction, in order to change the drug storage space in the drug storage tank, and the first direction is the arrangement direction of the bottom and top of the drug storage tank;
the two conductive push rods are respectively connected to the side of the push rod piston away from the injection port, and each conductive push rod extends along the first direction; the two conductive push rods are parallel and spaced apart;
the injection detection module comprises a control circuit board, two conductive contact springs, and a conductive module; the two conductive contact springs are spaced apart, and electrically connected to the control circuit board; the two conductive push rods are in a sliding contact with the two conductive contact springs;
the power module is used to drive the conductive module to move in the first direction, so that both ends of the conductive module are respectively in contact with the two conductive push rods; and
the control circuit board is used to detect the dosage injected into the drug storage tank based on the moving distance of the conductive module.

The patch type insulin pump provided by the present application is provided with an injection detection module, used to detect the dosage injected into the drug storage tank. Specifically, firstly, two conductive contact springs respectively connected to the control circuit board are provided to contact two conductive push rods, and a conductive module is provided; the conductive push rod always keeps in contact with the conductive contact springs in the moving process. When two conductive push rods are not in contact with the conductive module, the circuit formed among the control circuit board, conductive contact springs, conductive push rod, and conductive module is disconnected. However, when the conductive module moves to contact the conductive push rod, the circuit is closed and can generate continuous electrical signals. When the user injects medication into the drug storage tank, the push rod piston and conductive push rod move towards the conductive module under the thrust of the medication, and then control the circuit board to control the conductive module to move towards the conductive push rod until the two come into contact, thus generating an electrical signal. The control circuit board can calculate the moving distance of the conductive module based on the time of generating the electrical signal, and then calculate the moving distance of the push rod piston during the injection of medication when the push rod piston moves to contact with the conductive push rod when the push rod piston is in the initial state, thereby calculating the dosage injected by the user. The patch type insulin pump in the present application can detect the dosage injected by the user by provision of an injection detection module, ensuring accurate medication and improving the user experience.
In some possible embodiments, the power module comprises a lead screw, a sliding block, and a driving module;
the lead screw extends along the first direction, and the driving module is used to drive the lead screw to rotate around its own axis;
the sliding block is connected to the lead screw in a transmission way, so that when the lead screw rotates around its own axis, it drives the sliding block to move in the first direction; and
the conductive module is fixed to the sliding block.

In some possible embodiments, the power module further comprises a bracket, and the lead screw can be rotationally installed on the bracket around its own axis relative to the bracket;
the bracket is provided with guide holes corresponding to the two conductive push rods, each of which is threaded through the corresponding guide holes, and the lead screw is located between the two conductive push rods.

In some possible embodiments, the bracket is made of a non-conductive material; the conductive module is arranged around the circumference of the lead screw, and there is a gap between the conductive module and the lead screw.

In some possible embodiments, the driving module comprises a stepping motor.

In some possible embodiments, the conductive module is a conductive cloth, alternatively, the conductive module is made of metal.

In some possible embodiments, the conductive push rod is made of metal, and the conductive contact spring is made of metal.

In some possible embodiments, the control circuit board is further used to drive the conductive module to move to a predetermined position along the first direction according to a set injection amount.

In some possible embodiments, further comprising a buzzer reminder module, which is used to sound a buzzer alarm when the conductive module moves to the predetermined position.

In a second aspect, the present application provides an insulin injection system, comprising an operating device and the patch type insulin pump in any possible embodiment of the first aspect, wherein the control circuit board is in signal connection to the operating device for receiving signals sent by the operating device and controlling the operation of the power module based on the signals sent by the operating device.

### Brief Description of the Drawings

Fig. 1 is a schematic diagram of an overall structure of the patch type insulin pump in the embodiment of the present application;
Fig. 2 is a schematic diagram of an explosion structure of the patch type insulin pump in the embodiment of the present application;
Fig. 3 is a schematic diagram of a cross-sectional structure of the patch type insulin pump in the embodiment of the present application after being injected with medication by the user;
Fig. 4 is a schematic diagram of a cross-sectional structure of the patch type insulin pump in Fig. 3 when detecting the dosage injected; and
Fig. 5 is a schematic diagram of a cross-sectional structure of the insulin pump during dosage control in the embodiment of the present application.

In the drawings:
100-injection module; 110-drug storage tank; 111-injection port; 120-push rod piston; 130-conductive push rod; 200-power module; 210-stepping motor; 220-transmission module; 230-bracket; 231-first support frame; 232-second support frame; 233-connecting plate; 240-lead screw; 250-sliding block; 300-injection detection module; 310-control circuit board; 320-conductive contact spring; 330-conductive module.

### Detailed Description of the Preferred Embodiments

Technical solutions in the embodiments of the present invention will be described clearly and completely in combination with figures in the embodiments of the invention. Obviously, the described embodiments are only part, but not all, of the embodiments of the invention. All other embodiments obtained by those of ordinary skill in the art without creative work based on the embodiments of the present invention are within the scope of protection of the present invention.

The embodiment of the present application provides an insulin injection system, which comprises an operating device and a patch type insulin pump. The operating device can be used to control the patch type insulin pump for detecting the dosage injected into the patch type insulin pump and achieving automatic injection of the patch type insulin pump. In practical applications, the operating device can be, for example, an operating program set on a smartphone, and users can control the patch type insulin pump by clicking on different options in the operating program. The following will provide a detailed explanation of the above patch type insulin pump in conjunction with specific embodiments.

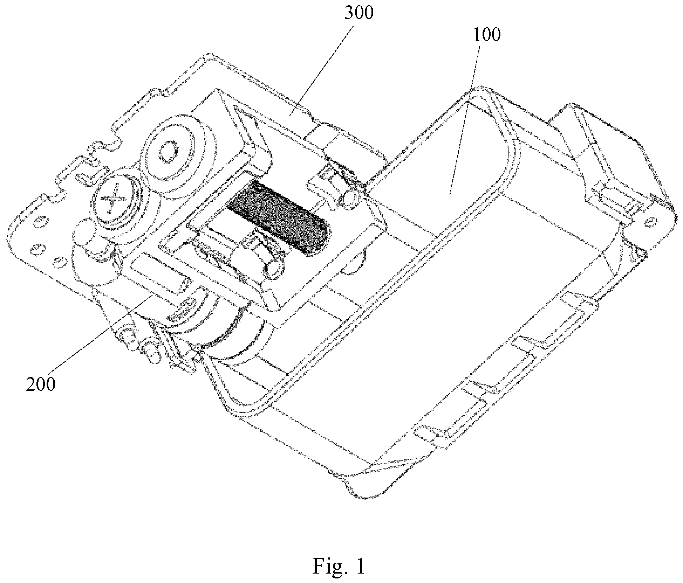

Referring to Fig. 1, the patch type insulin pump in this embodiment can comprise an injection module 100, a power module 200, and an injection detection module 300, wherein the user can inject the medication from the drug bottle into the injection module 100, and then inject the medication into the body through the injection module 100. The power module 200 and the injection detection module 300 can be used in conjunction to detect the injected dosage. The power module 200 can also be used to inject the medication from the injection module 100 into the user's body.

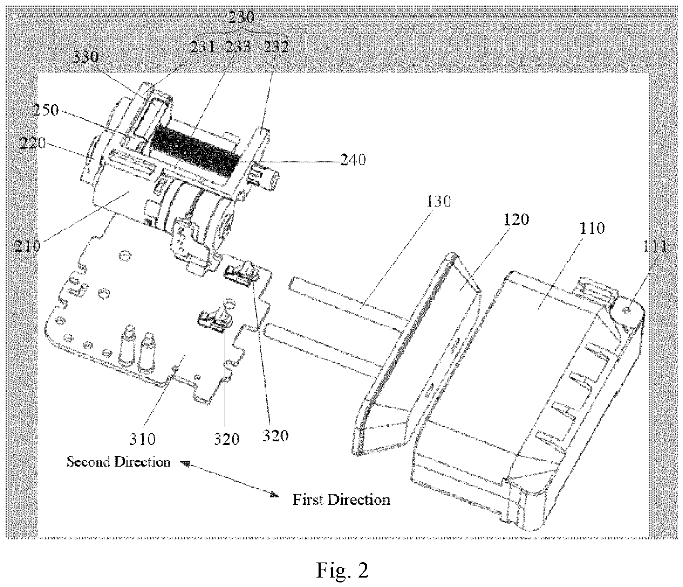

Specifically, referring to Fig. 2, the injection module 100 comprises a drug storage tank 110, a push rod piston 120, and two conductive push rods 130. The bottom of the drug storage tank 110 is provided with an injection port 111 that is internally connected to the drug storage tank 110. Users can inject the medication in the drug bottle into the drug storage tank 110 through the injection port 111. The top of the drug storage tank 110 is provided with an opening, and the push rod piston 120 is sealed on the opening. At this time, the bottom of the push rod piston 120 matches with the internal space of the drug storage tank 110 to form a drug storage space. The push rod piston 120 can also ensure that the medication in the drug storage space will not flow out from the opening. It is supposed that the first direction is the arrangement direction from the top to bottom of the drug storage tank 110, and suppose the second direction is the arrangement direction from the bottom to the top of the drug storage tank 110, and the push rod piston 120 can also move relative to the drug storage tank 110 along the first direction to change the size of the drug storage space.

The two conductive push rods 130 are respectively connected to the side of the push rod piston 120 away from the injection port 111, and each conductive push rod 130 extends along the first direction; moreover, the two conductive push rods 130 are spaced apart; When the injection module 100 is in the initial state, the bottom of the push rod piston 120 contacts the bottom wall. When the user injects the medication into the drug storage tank 110 through the injection port 111, under the push of the medication, the push rod piston 120 moves away from the injection port 111, while driving the conductive push rod 130 to move away from the injection port 111.

Continuing with reference to Fig. 2, the injection detection module 300 comprises a control circuit board 310, two conductive contact springs 320, and a conductive module 330. The two conductive contact springs 320 are electrically connected to the control circuit board, and exemplarily, the conductive contact springs 320 can be fixed to the control circuit board 310 by soldering, screw fastening fixing, riveting, laser welding, or connecting by means of electrically connecting elements. The two conductive contact springs are spaced apart, and the two conductive contact springs 320 are respectively in sliding contact with the two conductive push rods, that is to say, each conductive push rod 130 always keep in contact with the conductive contact springs 320 in the process of moving along the first direction. The conductive contact spring 320 can have a certain elasticity. When the conductive push rod 130 moves relative to the conductive contact spring 320, the conductive contact spring 320 can generate a certain amount of deformation and accumulate force, so that the conductive contact spring 320 can always keep in contact with the conductive push rod 130.

The conductive module 330 is located on the side of the conductive push rod 130 away from the push rod piston 120. The conductive module 330 is connected to the power module 200, and the power module 200 can drive the conductive module 330 to move along the first direction until the conductive module 330 contacts both conductive push rods 130 at the same time. At this time, the control circuit board 310, two conductive contact springs 320, two conductive push rods 130, and the conductive module 330 form a closed circuit, and the current in the circuit can flow normally, thereby generating continuous electrical signals. The control circuit board 310 is in signal connection with the power module 200, and the control circuit board can receive electrical signals and generate signals for controlling the working status of the power module 200, thereby driving the power module 200 to start or stop working.

Continuing with reference to Fig. 2, the power module 200 comprises a driving module (refer to reference sign 210 in Fig. 2), a transmission module 220, a bracket 230, a lead screw 240, and a sliding block 250, wherein the lead screw 240, the drive module, and the transmission module 220 can all be installed on the bracket 230, so that the overall structure of the power module 200 is stable. Specifically, the bracket 230 may comprise a first support frame 231 and a second support frame 232 which are arranged in parallel, and a connecting plate 233 connected between the first support frame 231 and the second support frame 232, so that the bracket 230 is a U-shaped structure as a whole. The driving module can be fixed on the connecting plate 233, and the transmission module 220 is fixed on the side of the first support frame 231 away from the second support frame 232. The lead screw 240 extends along the first direction, that is, the axis direction of the lead screw 240 is consistent with the first direction, and both ends of the lead screw 240 are respectively threaded through the first support frame 231 and the second support frame 232.

Furthermore, the sliding block 250 can be sleeved on the lead screw 240, and connected to the lead screw 240 in a transmission way. In addition, the conductive module 330 can be fixed on the side of the sliding block 250 towards the conductive push rod 130. In this embodiment, the driving module is connected to the transmission module 220 in a transmission way, and the transmission module 220 is connected to one end of the lead screw 240 threaded through the first support frame 231 in a transmission way. Therefore, the power output by the driving module can be transmitted to the lead screw 240 through the transmission module 220, thereby driving the lead screw 240 to rotate relative to the bracket 230 around its own axis. When the lead screw 240 rotates, it can drive the sliding block 250 to move along the first direction, thereby driving the conductive module 330 to move along the first direction.

The conductive module 330 is arranged around the circumference of the lead screw 240, and there is a gap between the conductive module 330 and the lead screw 240 to ensure that there is no contact between the conductive module 330 and the lead screw 240. For example, the conductive module 330 can be "back" or U-shaped, so that the lead screw 240 can pass through the hollow part of the conductive module 330 and be connected to the sliding block 250 in a transmission way.

The second support frame 232 is provided with guide holes corresponding to the two conductive push rods 130 (not shown in the figure), each of which can be threaded through the corresponding guide holes. At this time, the lead screw 240 is located between the two conductive push rods 130, and the lead screw 240 is spaced apart from each conductive push rod 130. When the conductive module 330 moves along the first direction, the conductive module 330 can avoid the lead screw 240 and make contact with the two conductive push rods 130, forming a closed circuit.

In the present embodiment, the conductive module 330 can be, for example, a conductive cloth, or the conductive module 330 is made of metal to ensure good conductivity of the conductive module 330. Similarly, the conductive push rod 130 can be made of metal. While ensuring the good conductivity of the conductive push rod 130, it can also improve its strength, ensuring that the conductive push rod 130 will not deform during movement and will not affect contact with the conductive module 330. Similarly, the conductive contact spring 320 can also be made of metal to ensure good conductivity between the conductive push rod 130 and the conductive contact spring 320.

It is worth mentioning that both the bracket 230 and sliding block 250 in this embodiment are made of non-conductive materials. When the conductive push rod 130 moves along the first direction, the conductive push rod 130 will not generate electrical signals due to contact with the bracket 230 or sliding block 250, thereby interfering with the operation of the control circuit board 310. Therefore, the patch-type insulin pump in this embodiment can not only detect the amount of medicine, but can also improve the detection accuracy of the injected medicine amount.

For the convenience of calculating the dosage injected into the drug storage tank 110, as shown in Fig. 2, the driving module can be a stepping motor 210. At this time, the moving distance of the conductive module 330 can be calculated by the total number of revolutions of the output shaft of the stepping motor 210, which is simple and convenient. For example, when the total moving distance of the conductive module 330 is 10.00mm, the stepping motor 210 needs to rotate 8000 times, so it is concluded that the displacement of the conductive module 330 is 0.00125mm after one rotation of the stepping motor 210. By deducing the number of rotations C of the stepping motor 210 as detected through the drive module, the total displacement of the conductive module 330 is L=C*0.00125mm. In addition, the drug storage space inside the drug storage tank 110 is of a regular three-dimensional shape, for example, the drug storage space is of a square or cylindrical structure, so that the dosage injected into the drug storage tank 110 can be calculated by the moving distance of the push rod piston 120. Specifically, when both the injection module 100 and the injection detection module 300 are in their initial state, the bottom of the push rod piston 120 contacts the inner wall of the drug storage tank 110, indicating that there is no medication in the drug storage tank 110, and the dosage stored in the drug storage tank 110 is 0. At this time, the conductive module 330 and the conductive push rod 130 are in a state of disengagement, and the conductive push rod 130 is located farthest from the conductive module 330. Then the conductive module 330 is driven to move towards the conductive push rod 130 and make contact with the conductive push rod 130. The distance for the conductive module 330 to move along the first direction is L. When the piston push rod is in the middle of the drug storage tank 110, it indicates that there is a certain amount of medication in the drug storage tank 110. At this time, when the conductive module 330 moves towards the conductive push rod 130 and comes into contact with the conductive push rod 130, the distance for the conductive module 330 to move along the first direction is L1. Therefore, the moving distance of the push rod piston 120 in the drug storage tank 110 is L2, that is, the height of the injected medication is h=L2=L-L1. At this time, the dosage injected can be calculated by formula based on the shape of the drug storage tank 110. For example, the cross-sectional area of the drug storage tank 110 is S and the height of the injected medication is L2. The total volume V of the injected medication is obtained through the volume formula V=S*h=S*L2.

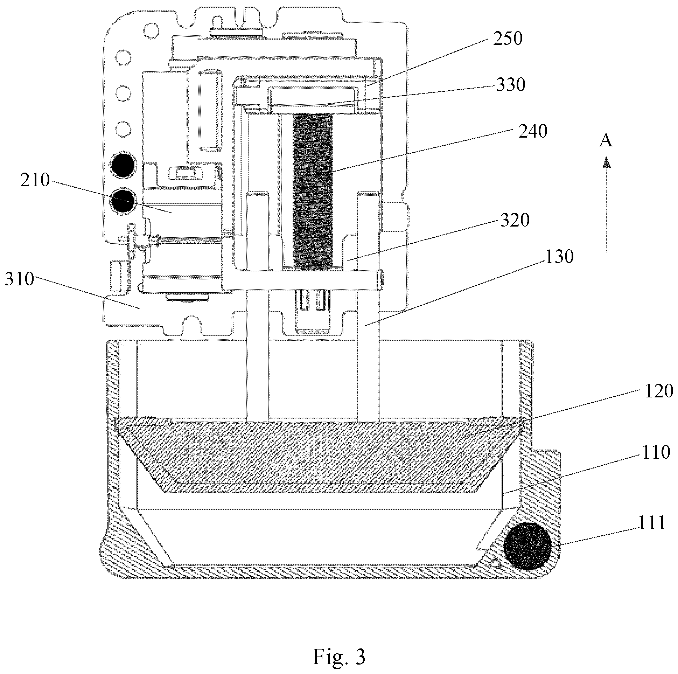

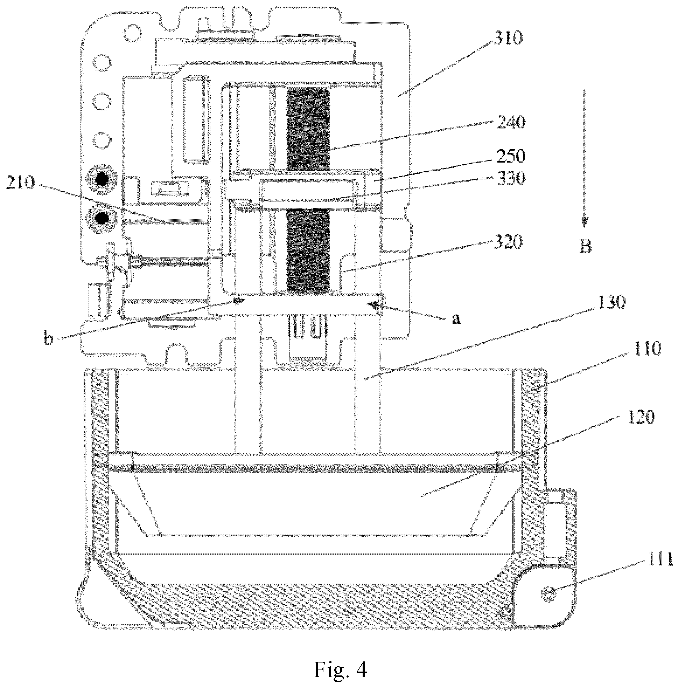

In specific implementation, when the user downloads the corresponding operating program on the smartphone, the signal connection between the operating program and the control circuit board 310 can be achieved through either wired or wireless connection, such as Bluetooth connection. Referring to Figs. 3 and 4 together, with both the injection module 100 and the injection detection module 300 in their initial states, the user injects the medication from the drug bottle into the drug storage tank 110 through the injection port 111. The push rod piston 120 moves away from the injection port 111 along the direction A in Fig. 3 under the thrust of the medication, until the user completes the injection. The push rod piston 120 stops moving and remains stationary. At this point, the user can click on the "injection completed" option in the operating program. The operating program will send the generated corresponding signal M to the control circuit board 310. The control circuit board 310 will generate a signal N that drives the conductive module 330 to move towards the conductive push rod 130 by using the power module based on the received signal M, and send this signal N to the stepping motor 210. Upon the receipt of the signal N, the stepping motor 210 starts working, drives the sliding block 250 and the conductive module 330 to move towards the conductive push rod 130 along the B direction in Fig. 4, until both ends of the conductive module 330 come into contact with the two conductive push rods 130 respectively. At this point, the current at point a in Fig. 4 will flow along the conductive push rod 130 and conductive module 330 to point b, generating a continuous electrical signal P. The control circuit board 310 receives the electrical signal P and generates a signal Q to control the stepping motor 210 to stop working based on the electrical signal P. The signal Q is then sent again to the stepping motor 210 to stop working. The control circuit board 310 can calculate the total number of revolutions of the output shaft within the time interval between sending an N signal to the stepping motor 210 and receiving a P signal to stop, and calculate the moving distance of the conductive module 330 along the B direction, thereby detecting the dosage injected into the drug storage tank 110. Finally, the control circuit board 310 sends the injected dosage generation signal to the operating program and displays it on the smartphone, making it easier for users to view the injected dosage.

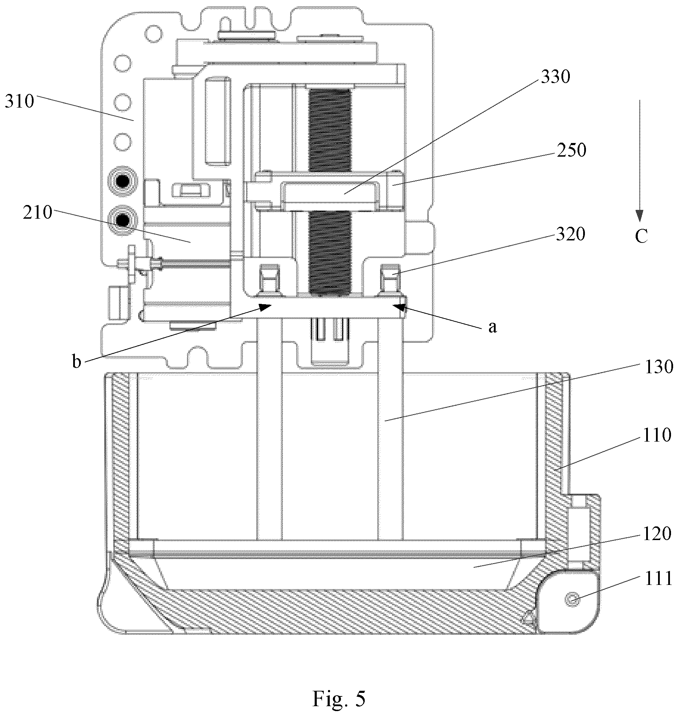

In some embodiments, the control circuit board 310 in this embodiment can also perform dosage control, that is, controlling the dosage injected by the user to enable precise medication. Specifically, as shown in Fig. 5, the control circuit board 310 can obtain the total rotation times C=L/0.00125 of the stepping motor based on a predetermined amount of medication Vml, a known cross-sectional area of the drug storage tank 110 of S, and a calculated displacement L=V/S of the conductive module 330. Then, based on the moving distance h2 of the conductive module 330 moving towards the conductive push rod 130 and coming into contact with the conductive push rod 130 when both the injection module 100 and the injection detection module 300 are in their initial state, the distance h of the conductive module 330 moving from the initial position to the predetermined position is calculated as h2-hl. That is to say, in the present embodiment, the conductive module 330 can be first moved towards the conductive push rod 130 to a predetermined position, and then the medication can be injected into the drug storage tank 110. Under the thrust of the medication, when the conductive push rod 130 moves to contact with the conductive module 330, the conductive push rod 130 cannot move further. At this time, the injection stops, ensuring that the dosage injected into the drug storage tank 110 is constant.

Based on this, the patch type insulin pump in this embodiment can also be provided with a buzzer reminder module (not shown in the figure), which is in signal connection to the control circuit board 310. When the conductive module 330 contacts the conductive push rod 130, the control circuit board 310 can generate a signal T. Upon receipt of the signal T, the buzzer reminder module can sound a buzzer alarm to remind the user to complete the injection.

In specific implementation, in conjunction with Fig. 5, users can first input the predetermined dosage on the operating program. The operating program generates the corresponding signal U from the predetermined dosage and sends the signal U to the control circuit board 310. The control circuit board 310 calculates the distance h that the conductive module 330 needs to move based on the predetermined dosage, generates a signal V, and sends the signal V to the stepping motor 210 to drive it to work, causing the conductive module 330 to move along the C direction in Fig. 5 to the predetermined position H. At this point, the stepping motor 210 stops working. Then, the user injects the medication into the drug storage tank 110 through the injection port 111. The medication pushes the push rod piston 120 towards the conductive module 330 until it comes into contact with the conductive module 330. At this time, the current at point a will flow along the conductive push rod 130 and the conductive module 330 to point b, generating a continuous electrical signal W. The control circuit board 310 receives this electrical signal W and generates a signal T based on the electrical signal W, causing the buzzer reminder module to sound an alarm to remind the user that the required volume of medication has been injected. At the same time, the user cannot push the conductive module 330 to move, ensuring that the user cannot inject more dosage.

It will be apparent to those skilled in the art that various amendments and variations may be made to the present invention without departing from the spirit and scope of the present invention. In this way, if these amendments and variations of the present invention are within the ranges of the claims of the present invention and equivalent technologies thereof, the present invention has also intended to contain those amendments and modifications.

## Claims

1. A patch type insulin pump, **characterized by** comprising an injection module, a power module and an injection detection module,
wherein the injection module comprises a drug storage tank, a push rod piston, and two conductive push rods; a bottom of the drug storage tank is provided with an injection port, and a top of the drug storage tank is provided with an opening; a push rod piston is sealed on the opening, and the push rod piston is configured to move relative to the drug storage tank along a first direction, in order to change the drug storage space in the drug storage tank, and the first direction is the arrangement direction of the bottom and top of the drug storage tank;
wherein the two conductive push rods are respectively connected to the side of the push rod piston away from the injection port, and each conductive push rod extends along the first direction; the two conductive push rods are parallel and spaced apart;
wherein the injection detection module comprises a control circuit board, two conductive contact springs, and a conductive module; the two conductive contact springs are spaced apart, and electrically connected to the control circuit board; the two conductive push rods are in a sliding contact with the two conductive contact springs;
wherein the power module is used to drive the conductive module to move in the first direction, so that both ends of the conductive module are respectively in contact with the two conductive push rods; and
wherein the control circuit board is used to detect the dosage injected into the drug storage tank based on the moving distance of the conductive module.

2. The patch type insulin pump according to claim 1, **characterized in that**, the power module comprises a lead screw, a sliding block, and a driving module;
wherein the lead screw extends along the first direction, and the driving module is used to drive the lead screw to rotate around its own axis;
wherein the sliding block is connected to the lead screw in a transmission way, so that when the lead screw rotates around its own axis, it drives the sliding block to move in the first direction; and
wherein the conductive module is fixed to the sliding block.

3. The patch type insulin pump according to claim 2, **characterized in that**, the power module further comprises a bracket, and the lead screw is configured to be rotationally installed on the bracket around its own axis relative to the bracket; and
wherein the bracket is provided with guide holes corresponding to the two conductive push rods, each of which is threaded through the corresponding guide holes, and the lead screw is located between the two conductive push rods.

4. The patch type insulin pump according to claim 3, **characterized in that**, the bracket is made of a non-conductive material; the conductive module is arranged around the circumference of the lead screw, and there is a gap between the conductive module and the lead screw.

5. The patch type insulin pump according to claim 2, **characterized in that**, the driving module comprises a stepping motor.

6. The patch type insulin pump according to claim 1, **characterized in that**, the conductive module is a conductive cloth or the conductive module is made of metal.

7. The patch type insulin pump according to claim 1, **characterized in that**, the conductive push rod is made of metal, and the conductive contact spring is made of metal.

8. The patch type insulin pump according to claim 1, **characterized in that**, the control circuit board is further used to drive the conductive module to move to a predetermined position along the first direction according to a set injection amount.

9. The patch type insulin pump according to claim 8, **characterized by** further comprising a buzzer reminder module, which is used to sound a buzzer alarm when the conductive module moves to the predetermined position.

10. An insulin injection system, **characterized by** comprising an operating device and the patch type insulin pump according to any of claims 1~9, wherein the control circuit board is in signal connection to the operating device for receiving signals sent by the operating device and controlling the operation of the power module based on the signals sent by the operating device.
